(19) Europäisches Patentamt
European Patent Office
Office européen des brevets

(11) **EP 1 806 099 A1**

(12) **EUROPEAN PATENT APPLICATION**
published in accordance with Art. 158(3) EPC

(43) Date of publication:
**11.07.2007 Bulletin 2007/28**

(51) Int Cl.:
**A61B 8/08** (2006.01)

(21) Application number: **05795508.0**

(22) Date of filing: **18.10.2005**

(86) International application number:
**PCT/JP2005/019088**

(87) International publication number:
**WO 2006/043528 (27.04.2006 Gazette 2006/17)**

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **19.10.2004 JP 2004303872**

(71) Applicant: **MATSUSHITA ELECTRIC INDUSTRIAL CO., LTD.**
**Osaka 571-8501 (JP)**

(72) Inventors:
• **KATO, Makoto**
**Matsushita Electric Industrial Co., Ltd**
**Shirumi 1-chome, Chou-Ku**
**Osaka-shi Osaka 5406319 (JP)**

• **HAGIWARA, Hisashi**
**Matsushita Electric Industrial Co., Ltd**
**Shirumi 1-chome, Chou-Ku**
**Osaka-shi Osaka 5406319 (JP)**

• **TANNAKA, Yoshinao**
**Matsushita Electric Industrial Co., Ltd**
**Shirumi 1-chome, Chou-Ku**
**Osaka-shi Osaka 5406319 (JP)**

(74) Representative: **Grünecker, Kinkeldey, Stockmair & Schwanhäusser**
**Anwaltssozietät**
**Maximilianstrasse 58**
**80538 München (DE)**

(54) **ULTRASONOGRAPHIC DEVICE AND ULTRASONOGRAPHIC DEVICE CONTROL METHOD**

(57) The ultrasonic diagnostic apparatus of the present invention includes: a transmitting section **14** for driving an ultrasonic probe **13** to send an ultrasonic transmitted wave to an organism's tissue; a receiving section **15** for amplifying an ultrasonic reflected wave, produced by getting the transmitted wave reflected by the tissue and received by the ultrasonic probe **13,** to generate a received signal; a frame calculating section **19** for calculating shape measured values of the tissue based on the received signal and figuring out a spatial distribution frame, representing the spatial distribution of the shape measured values and/or property measured values of the organism every cardiac cycle, based on the shape measured values of the tissue; a difference calculating section **19** for calculating a difference between the shape measured values or property measured values of two of the spatial distribution frames calculated every cardiac cycle; a storage section for storing the shape measured values, property measured values and/or difference; and a display section **21** for presenting the frames thereon.

FIG.1

## Description

### TECHNICAL FIELD

The present invention relates to an ultrasonic diagnostic apparatus. More particularly, the present invention relates to an ultrasonic diagnostic apparatus for inspecting the property of a vital tissue and a method for controlling such an ultrasonic diagnostic apparatus.

### BACKGROUND ART

Recently, the number of people suffering from various circulatory system diseases, including heart infarction and brain infarction, has been on the rise, thus making it more and more urgent to prevent and treat these diseases.

The pathopoiesis of heart or brain infarction is closely correlated to arterial sclerosis. More specifically, if an atheroma is created on the arterial wall or if no arterial cells are produced anymore due to various factors such as elevated blood pressure, then the artery loses its elasticity to become hard and fragile. Also, if the blood vessel is clogged up where the atheroma has been created or if a vascular tissue covering the atheroma has ruptured, then the atheroma will move itself into the blood vessel to clog up the artery elsewhere or to rupture the hardened portions of the artery. As a result, these diseases are caused. That is why it is important to diagnose the arterial sclerosis as early as possible to prevent or treat these diseases.

In the prior art, the lesion of arterial sclerosis is diagnosed by directly observing the inside of the blood vessel with a vascular catheter. However, this diagnosis needs to be carried out with a vascular catheter inserted into the blood vessel of a patient, thus imposing a heavy load on him or her. For that reason, the vascular catheter observation is usually adopted to locate the lesion of arterial sclerosis in a patient who is already known to suffer from that disease but has never been used to make a medical checkup on a supposedly healthy person.

A checkup may be easily made without imposing excessively heavy load on a patient if the index of cholesterol, which is one of major causes of arterial sclerosis, or the blood pressure is measured. However, none of these values directly indicates the degree of advancement of arterial sclerosis.

Also, if the arterial sclerosis can be diagnosed early enough to administer some medicine to its patient, then the disease can be treated effectively. However, it is said that once the arterial sclerosis has advanced to a certain degree, the farther advancement of that disease can be checked with the administration of medicine but it is difficult to repair the hardened artery completely.

For these reasons, a method or apparatus for diagnosing the arterial sclerosis at an early stage of its advancement without imposing too much load on its patient is now in high demand.

Meanwhile, an ultrasonic diagnostic apparatus or an X-ray diagnostic apparatus has been used in the prior art as a noninvasive medical apparatus that imposes only a light load on a person under test. Specifically, by irradiating the testee with an ultrasonic wave or an x-ray that has been produced externally, shape information or information about the variation in the shape of his or her internal body with time can be acquired without causing pain to him or her. When the information about the variation with time (i.e., mobility information) in the shape of an object under test in his or her body can be obtained, the property information of the object can be obtained. That is to say, the vascular elastic property of the organism can be known and the degree of advancement of the arterial sclerosis can be detected directly.

Among other things, the ultrasonic diagnosis is superior to the X-ray diagnosis because the ultrasonic diagnosis can be made just by putting an ultrasonic probe on a person under test. That is to say, in the ultrasonic diagnosis, there is no need to administer a contrast medium to the person under test and there is no concern about potential X-ray exposure, either.

Besides, some ultrasonic diagnostic apparatuses can recently have significantly improved measuring accuracy thanks to remarkable advancement of electronic technologies. As a result, ultrasonic diagnostic apparatuses for measuring the very small motion of a vital tissue have been developed. For example, according to the technique disclosed in Patent Document No. 1, vibration components of a vascular motion, having an amplitude of several micrometers and a frequency of as high as several hundreds of Hz, can be measured accurately. Thus, it was reported that the thickness variation or strain of the vascular wall could be measured highly accurately on the order of several micrometers.

By adopting such a high-accuracy measuring technique, the two-dimensional distribution of the elastic property of the arterial wall can be plotted in detail. For example, Non-Patent Document No. 1 shows an example of presenting the two-dimensional distribution of the elasticity of the iliac bone arterial vascular wall as an image superimposed on a B-mode tomogram. The hardness of the arterial wall is not uniform but has some distribution. That is why in diagnosing the arterial sclerosis, it is important to understand properly the local distribution of the elasticity, which is a characteristic quantity showing the degree of advancement of the arterial sclerosis.

Patent Document No. 1: Japanese Patent Application Laid-Open Publication No. 10-5226
Non-Patent Document No. 1: Hiroshi Kanai et al., "Elasticity Imaging of Atheroma with Transcutaneous Ultrasound Preliminary Study", Circulation, Vol. 107, pp. 3018-3021, 2003

## DISCLOSURE OF INVENTION

## PROBLEMS TO BE SOLVED BY THE INVENTION

As the heart beats, a circulatory tissue such as the artery repeatedly displaces, shrinks or dilates. That is why to measure the strain or elastic property of a circulatory tissue using an ultrasonic diagnostic apparatus, the measurements is preferably taken once every cardiac cycle. That is to say, the distribution of strain or elastic property is preferably obtained every cardiac cycle. The elastic property thus measured, however, often changes one cardiac cycle after another. The reasons are that a cardiac cycle is not constant strictly speaking, that the operator of an ultrasonic probe or a subject will make unnecessary motions during the measurements, and that the measurements tend to be affected by various sorts of noise according to the measuring environment. Due to these factors, the elastic property measured probably has some variations and it is difficult to determine whether the result of measurement has accurate values or not.

In order to overcome the problems described above, an object of the present invention is to provide an ultrasonic diagnostic apparatus that can determine whether or not the measured values have varied due to those factors and also provide a method for controlling an ultrasonic diagnostic apparatus.

## MEANS FOR SOLVING THE PROBLEMS

An ultrasonic diagnostic apparatus according to the present invention includes: a transmitting section for driving an ultrasonic probe that sends out an ultrasonic transmitted wave toward a tissue of an organism; a receiving section for amplifying an ultrasonic reflected wave to generate a received signal, the ultrasonic reflected wave being produced by getting the ultrasonic transmitted wave reflected by the tissue of the organism and being received by the ultrasonic probe; a frame calculating section for calculating shape measured values of the tissue based on the received signal and figuring out a spatial distribution frame, representing the spatial distribution of the shape measured values and/or property measured values of the organism every cardiac cycle, based on the shape measured values of the tissue; a difference calculating section for calculating a difference between the shape measured values or the property measured values of two frames that have been selected from the spatial distribution frames calculated every cardiac cycle; a storage section for storing at least one of the shape measured values, the property measured values and the difference; and a display section for presenting the spatial distribution frames thereon.

In one preferred embodiment, the difference calculating section calculates the difference between the newest and previous spatial distribution frames.

In another preferred embodiment, the difference calculating section calculates a number (N-1) of differences between the newest spatial distribution frame and previous (N-1) consecutive spatial distribution frames and further calculates one characteristic quantity, representing the degree of variation among a number N of spatial distribution frames, based on the (N-1) differences.

In this particular preferred embodiment, the difference calculating section calculates the difference between two spatial distribution frames that are continuous with each other on the time axis.

In a specific preferred embodiment, the difference calculating section updates the characteristic quantity every cardiac cycle.

In another preferred embodiment, the display section presents the difference.

In still another preferred embodiment, the display section presents at least one of the difference and the characteristic quantity.

In yet another preferred embodiment, the difference calculating section generates image information based on the difference and the display section presents the image information thereon.

In yet another preferred embodiment, the ultrasonic diagnostic apparatus further includes an acoustic transducer and the difference calculating section generates audio information based on the difference and the acoustic transducer outputs the audio information.

In yet another preferred embodiment, the difference calculating section generates the image information based on at least one of the difference and the characteristic quantity and the display section presents the image information thereon.

In yet another preferred embodiment, the ultrasonic diagnostic apparatus further includes an acoustic transducer, the difference calculating section generates audio information based on at least one of the difference and the characteristic quantity and the acoustic transducer outputs the audio information.

In yet another preferred embodiment, the difference calculating section compares either the difference or the characteristic quantity to a predetermined value. According to a result of the comparison, the frame calculating section updates the spatial distribution frame to be presented on the display section.

In yet another preferred embodiment, the difference calculating section finds a difference or a characteristic quantity, showing the smallest variation between spatial distribution frames, and the display section presents the spatial distribution frame associated with the difference or the characteristic quantity that has been found.

In yet another preferred embodiment, the difference is at least one of the average, the average of the absolute values, the sum, the sum of the absolute values, the variance, the standard deviation, the root mean square, and the difference between the maximum and minimum values of either the shape measured values or the property measured values in two frames that have been selected from the multiple spatial distribution frames.

In yet another preferred embodiment, the characteristic quantity is at least one of the average, the sum, the variance, the standard deviation, the root mean square, and the difference between the maximum and minimum values of the (N-1) differences.

In yet another preferred embodiment, the shape measured value represents a variation in the maximum thickness of the organism's tissue.

In yet another preferred embodiment, the property measured value is at least one of the strain and the elastic property of the organism's tissue.

An ultrasonic diagnostic apparatus controlling method according to the present invention is a method for controlling an ultrasonic diagnostic apparatus using a control section of the apparatus. The method includes the steps of: (a) sending out an ultrasonic transmitted wave from an ultrasonic probe and receiving an ultrasonic reflected wave to generate a received signal, the ultrasonic reflected wave being produced by getting the ultrasonic transmitted wave reflected by a tissue of an organism; (b) calculating shape measured values of the tissue based on the received signal and figuring out a spatial distribution frame, representing the spatial distribution of the shape measured values and/or property measured values of the organism every cardiac cycle, based on the shape measured values of the tissue; (c) calculating a difference between the shape measured values or the property measured values of two frames that have been selected from the spatial distribution frames calculated every cardiac cycle; and (d) presenting the spatial distribution frames.

In one preferred embodiment, the step (c) includes calculating the difference between the newest and previous spatial distribution frames.

In another preferred embodiment, the step (c) includes calculating a number (N-1) of differences between the newest spatial distribution frame and previous (N-1) consecutive spatial distribution frames and further calculating one characteristic quantity, representing the degree of variation among a number N of spatial distribution frames, based on the (N-1) differences.

In this particular preferred embodiment, the step (c) includes calculating the difference between two spatial distribution frames that are continuous with each other on the time axis.

In still another preferred embodiment, the step (c) includes updating the characteristic quantity every cardiac cycle.

In yet another preferred embodiment, the method further includes the step (e1) of presenting the difference.

In yet another preferred embodiment, the method further includes the step (e2) of presenting at least one of the difference and the characteristic quantity.

In yet another preferred embodiment, the step (e1) includes generating image information based on the difference and presenting the image information.

In yet another preferred embodiment, the method further includes the step (e3) of generating audio information based on the difference and outputting the audio information from an acoustic transducer.

In yet another preferred embodiment, the step (e2) includes generating the image information based on at least one of the difference and the characteristic quantity and presenting the image information.

In yet another preferred embodiment, the method further includes the step (e4) of generating audio information based on at least one of the difference and the characteristic quantity and outputting the audio information from an acoustic transducer.

In yet another preferred embodiment, the step (c) includes comparing either the difference or the characteristic quantity to a predetermined value, and the step (d) includes updating the spatial distribution frame to present according to a result of the comparison.

In yet another preferred embodiment, the step (c) includes finding a difference or a characteristic quantity showing the smallest variation between spatial distribution frames, and the step (d) includes presenting the spatial distribution frame associated with the difference or the characteristic quantity that has been found.

In yet another preferred embodiment, the difference is at least one of the average, the average of the absolute values, the sum, the sum of the absolute values, the variance, the standard deviation, the root mean square, and the difference between the maximum and minimum values of either the shape measured values or the property measured values in two frames that have been selected from the multiple spatial distribution frames.

In yet another preferred embodiment, the characteristic quantity is at least one of the average, the sum, the variance, the standard deviation, the root mean square, and the difference between the maximum and minimum values of the (N-1) differences.

In yet another preferred embodiment, the shape measured value represents a variation in the maximum thickness of the organism's tissue.

In yet another preferred embodiment, the property measured value is at least one of the strain and the elastic property of the organism's tissue.

## EFFECTS OF THE INVENTION

According to the present invention, a frame-by-frame difference is calculated between the greatest thickness differences, strains, or elastic properties, which are figured out based on the location information or motion information of an arbitrary area of a vital tissue using ultrasonic waves. This difference shows the degree of variation of the data that forms a frame. Alternatively, a number of such differences are calculated and a characteristic quantity showing the degree of variation of the frame is also produced. As a result, the stability of measurements can be evaluated based on either the difference or the characteristic quantity, and an accurate shape or property of the vital tissue can be measured. In addition, by presenting the shape or the property using the difference or the characteristic quantity, the results of measurements can have higher degree of reliability and can also be presented visually.

## BRIEF DESCRIPTION OF DRAWINGS

FIG. **1** is a block diagram showing an arrangement for a situation where an ultrasonic diagnostic apparatus according to the present invention is used to inspect the tissue and property of a vascular wall.
FIG. **2** is a block diagram showing a first preferred embodiment of an ultrasonic diagnostic apparatus according to the present invention.
FIG. **3** is a block diagram showing the detailed configuration of the computing section of the ultrasonic diagnostic apparatus shown in FIG. **2.**
FIG. **4** schematically illustrates an ultrasonic beam propagating through a vascular wall and respective measuring points.
FIG. **5** shows relations between measuring points and the tissue in question, of which the elasticity needs to be calculated.
FIG. **6(a)** schematically illustrates an ROI that is defined on a vascular wall image and FIG. **6(b)** schematically illustrates a spatial distribution frame presented on the display section.
FIG. **7** schematically shows how spatial distribution frames, the differences between the spatial distribution frames and the characteristic quantity of the differences correlate with each other.
FIG. **8** is a flowchart showing how an ultrasonic diagnostic apparatus according to a second preferred embodiment of the present invention operates.
FIG. **9** is a graph schematically showing the differences calculated during measurements in the second preferred embodiment.
FIG. **10** is a flowchart showing how an ultrasonic diagnostic apparatus according to a third preferred embodiment of the present invention operates.
FIG. **11** is a graph schematically showing the differences calculated during measurements in the third preferred embodiment.

## DESCRIPTION OF REFERENCE NUMERALS

**1** extravascular tissue
**2** body surface
**3** blood vessel
**4** vascular anterior wall
**5** blood

**11** ultrasonic diagnostic apparatus
**12** blood pressure manometer
**13** ultrasonic probe
**14** transmitting section
**15** receiving section
**16** time delay control section
**17** phase detecting section
**18** filter section
**19** computing section
**20** computed data storage section
**21** display section
**22** electrocardiograph
**31** shape measured value calculating section
**32** property measured value calculating section
**33** difference calculating section
**34** frame calculating section

## BEST MODE FOR CARRYING OUT THE INVENTION

An ultrasonic diagnostic apparatus according to the present invention calculates greatest thickness differences, which are shape measured values of respective portions of a vital tissue as the object of measurement, or strains and elastic properties, which are property measured values, and figures out the two-dimensional distribution thereof as a frame every cardiac cycle. Hereinafter, it will be described how the ultrasonic diagnostic apparatus of the present invention works in figuring out the two-dimensional distribution of the elastic properties of a vascular wall as an example.

(EMBODIMENT 1)

Hereinafter, a first preferred embodiment of an ultrasonic diagnostic apparatus according to the present invention will be described.

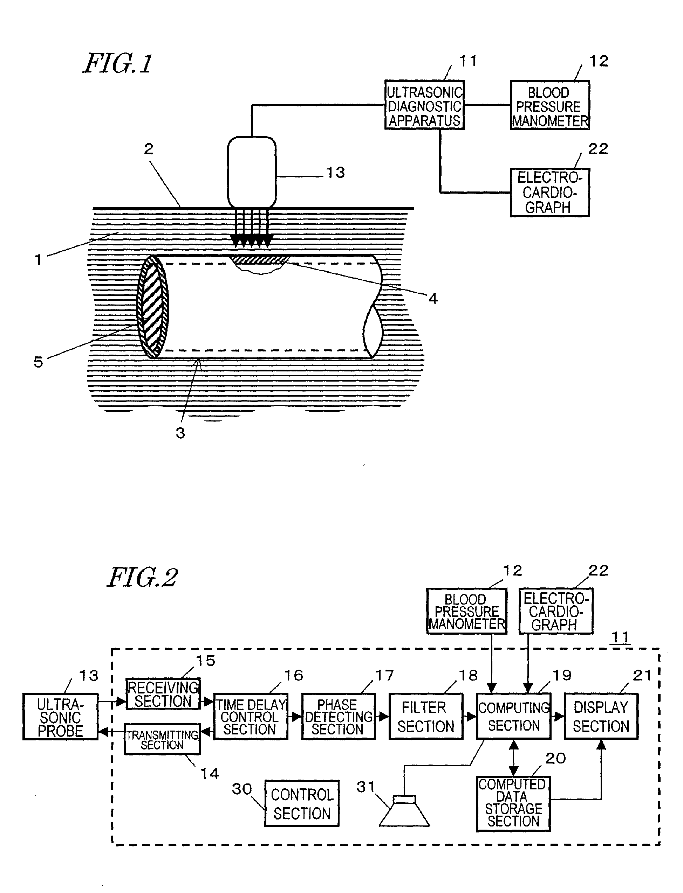

FIG. **1** is a block diagram showing an arrangement for a situation where the ultrasonic diagnostic apparatus **11** of this preferred embodiment is used to inspect the tissue and property of a vascular wall. An ultrasonic probe **13,** connected to the ultrasonic diagnostic apparatus **11,** is held in close contact with the body surface **2** of a person under test and transmits an ultrasonic wave into a body tissue inside an extravascular tissue **1.** The transmitted ultrasonic wave is reflected by a blood vessel **3** and blood **5,** scattered, and only a portion of it comes back to, and is received as an echo (i.e., the ultrasonic reflected wave) by, the ultrasonic probe **13.** The ultrasonic diagnostic apparatus **11** performs analysis and computations on the received signal, thereby acquiring the shape information and mobility information of the vascular anterior wall **4.** Also, a blood pressure manometer **12** is connected to the ultrasonic diagnostic apparatus **11** such that data about the blood pressure values of the person under measurement, collected by the blood pressure manometer **12,** is input to the ultrasonic diagnostic apparatus **11.** In accordance with the method disclosed in Patent Document No. 1, for example, the ultrasonic diagnostic apparatus **11** determines the instantaneous position of the object by a restricted minimum square method using both the amplitude and phase of a detection signal, thereby performing phase tracking highly accurately (where the magnitude of positional displacement has a measuring accuracy of about $\pm 0.2 \mu$ m) and measuring variations in the position and thickness of a very small spot on the vascular anterior wall **4** with time with sufficient precision. In addition, by using the blood pressure data obtained with the blood pressure manometer **12,** the ultrasonic diagnostic apparatus **11** can also evaluate the elastic property of a very small spot on the vascular anterior wall **4.** An electrocardiograph **22** is connected to the ultrasonic diagnostic apparatus **11,** which receives an electrocardiogram from the electrocardiograph **22** and uses it as a trigger signal that determines the timings of data acquisition and data resetting. The electrocardiograph **22** may be replaced with any other biomedical signal detecting means such as a phonocardiograph or a sphygmograph. In that case, a phonocardiogram or a sphygmogram may be used as a trigger signal instead of the electrocardiogram.

Hereinafter, the configuration and operation of the ultrasonic diagnostic apparatus **11** will be described in further detail. FIG. **2** is a block diagram showing a configuration for the ultrasonic diagnostic apparatus **11.** The ultrasonic diagnostic apparatus **11** includes a transmitting section **14,** a receiving section **15,** a time delay control section **16,** a phase detecting section **17,** a filter section **18,** a computing section **19,** a computed data storage section **20,** and a display section **21.** The ultrasonic diagnostic apparatus **11** further includes a control section **30** (including a microcomputer, for example) for performing an overall control on all of these sections.

The transmitting section **14** generates a predetermined drive pulse signal and outputs it to the ultrasonic probe **13.** An ultrasonic transmitted wave, transmitted by the ultrasonic probe **13** in response to the drive pulse signal, is reflected and scattered by a body tissue such as the blood vessel **3** to produce an ultrasonic reflected wave, which is then detected by the ultrasonic probe **13.** The frequency of the drive pulse that generates the ultrasonic wave is determined with the depth of the object of measurement and the velocity of the ultrasonic wave into consideration such that no ultrasonic pulses, adjacent to each other on the time axis, overlap with each other.

The receiving section **15** receives the ultrasonic reflected wave using the ultrasonic probe **13.** The receiving section **15** includes an A/D converting section and amplifies the ultrasonic reflected wave, thereby generating a received signal. And then the receiving section **15** further converts the received signal into a digital signal. The transmitting section **14** and receiving section **15** may be made of electronic components, for example.

The time delay control section **16** is connected to the transmitting section **14** and receiving section **15** in order to control the time delay of the drive pulse signal to be supplied from the transmitting section **14** to a group of ultrasonic vibrators in the ultrasonic probe **13.** In this manner, an ultrasonic beam of the ultrasonic transmitted wave to be transmitted from the ultrasonic probe **13** can have its acoustic line direction and depth of focus changed. Also, by controlling the time delay of the received signal that has been received by the ultrasonic probe **13** and then amplified by the receiving section **15,** the aperture size and depth of focus can be changed. The output of the time delay control section **16** is passed to the phase detecting section **17.**

The phase detecting section **17** detects the phase of the received signal, of which the time delay has been controlled by the time delay control section **16,** thereby splitting the signal into a real part signal and an imaginary part signal, which are then input to the filter section **18.** The filter section **18** filters out RF components, the components that have not been reflected by the object of measurement and other noise components. The phase detecting section **17** and filter section **18** may be implemented as either a software program or hardware components.

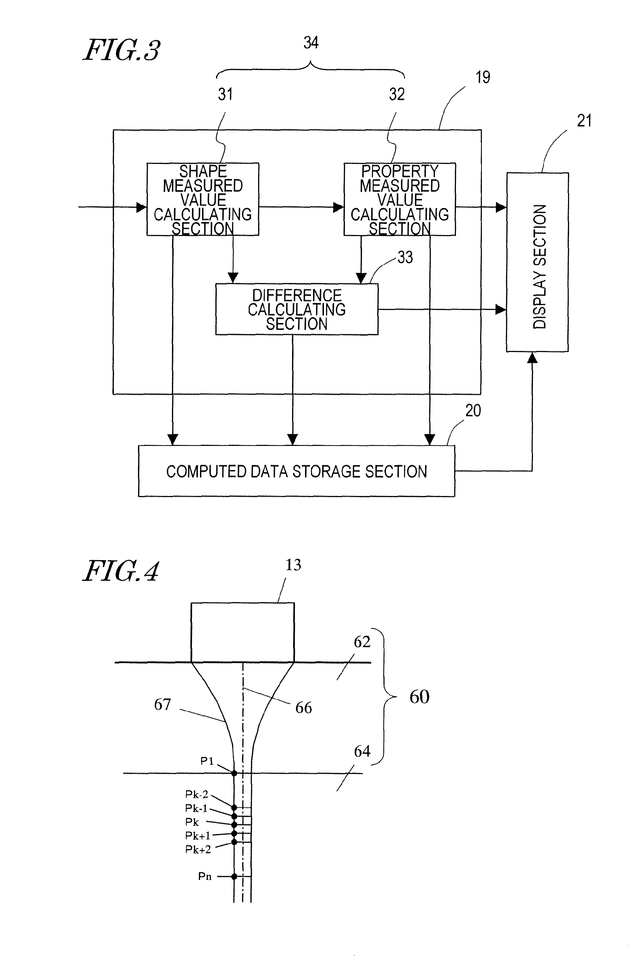

The real part signal and the imaginary part signal of the phase-detected received signal are input to the computing section **19.** FIG. **3** is a block diagram showing a detailed configuration for the computing section **19,** which includes a shape measured value calculating section **31,** an property measured value calculating section **32** and a difference calculating section **33.** The shape measured value calculating section **31** and property measured value calculating section **32** together form a frame calculating section **34.** The computing section **19** may be implemented as either a software program or as hardware components.

The shape measured value calculating section **31** calculates the motion velocities of the vital tissue at a plurality of measuring points based on the real-part and imaginary-part signals of the received signal and integrates the motion velocities together, thereby obtaining the magnitude of positional displacement (i.e., the magnitude of the displacement of a position with time). Then, based on the magnitude of positional displacement thus obtained, the shape measured value calculating section **31** calculates the variation in the thickness of the vital tissue (i.e., the magnitude of expansion/shrinkage) between the measuring points. Also, on receiving information about one cardiac cycle from the electrocardiograph **22,** the shape measured value calculating section **31** obtains the greatest thickness difference, which is the difference between the maximum and minimum thicknesses during one cardiac cycle, and the maximum thickness.

The property measured value calculating section **32** receives the greatest thickness difference and the maximum thickness value and calculates the strain of the vital tissue. Also, by using blood pressure data obtained from the blood pressure manometer **12,** the property measured value calculating section **32** figures out the elastic property of the tissue between measuring points.

The greatest thickness difference, strain or elastic property that has been obtained in this manner from the vital tissue is mapped on a measured region basis, thereby outputting a spatial distribution frame, representing the spatial distribution of the shape measured values or property measured values every cardiac cycle, to the display section **21.**

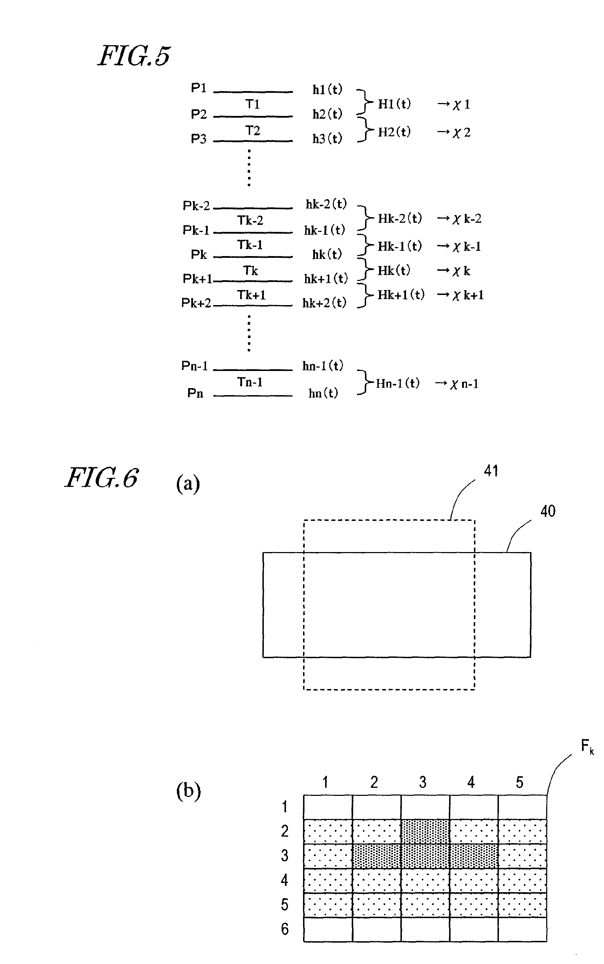

Hereinafter, it will be described in further detail with reference to FIGS. **4** and **5** how the frame calculating section **34** makes these calculations. FIG. **4** schematically illustrates an ultrasonic beam **67** propagating through an organism **60.** In FIG. **4,** a vascular wall **64** and a vital tissue **62** other than the blood vessel are shown. The ultrasonic transmitted wave, which has been sent out from the ultrasonic probe **13** that is put on the surface of the organism **60,** goes inside the organism **60.** The ultrasonic transmitted wave propagates as an ultrasonic beam **67** with a certain finite width inside of the organism **60.** In the meantime, a portion of the ultrasonic wave is either reflected or scattered by the vital tissue **62** and the vascular wall **64** back toward the ultrasonic probe **13** and received there as an ultrasonic reflected wave. The ultrasonic reflected wave is detected as a time series signal r(t). The closer to the ultrasonic probe **13** a portion of the tissue that has reflected the ultrasonic wave to produce the time series signal, the closer to the origin the signal is located on the time axis. The width (i.e., beam spot size) of the ultrasonic beam **67** can be controlled by changing the time delay.

A plurality of measuring points **P$_n$,** which are located on an acoustic line **66** (i.e., the center axis of the ultrasonic beam) on the vascular wall **62,** are arranged at regular intervals **L** in the order of $P_1$, $P_2$, $P_3$, ..., $P_k$, ... and $P_n$ (where n is natural number that is equal to or greater than three) where $P_1$ is a located closest to the ultrasonic probe. Supposing coordinates are defined in the depth direction with respect to the surface of the organism **60** as the origin such that the coordinates of the measuring points are represented by $Z_1$, $Z_2$, $Z_3$, ..., $Z_k$, ... and $Z_n$, an ultrasonic wave reflected from a measuring point **P$_k$** is located at $t_k = 2Z_k/c$ on the time axis, where c is the velocity of the ultrasonic wave in the organism. The reflected wave signal r(t) has its phase detected by the phase detecting section **17** and the phase-detected signal is split into a real part signal and an imaginary part signal, which are then passed through the filter section **18.** Under the restriction that the amplitude does not change, but only the phase and reflection spot change, between the reflected wave signal r(t) and another reflected wave signal r(t + Δt) obtained after a very small amount of time Δt, the shape measured value calculating section **31** of the computing section **10** calculates the phase difference by a minimum square method so as to minimize the waveform mismatch between the reflected wave signals r(t) and r(t + Δt). The motion velocity V$_n$(t) of the measuring point P$_n$ is derived from this phase difference and then integrated, thereby obtaining the mag-

nitude of positional displacement d$_n$(t).

FIG. **6** shows the relationship between the measuring point **P$_n$** and the tissue under test **T$_n$,** of which the elasticity needs to be calculated. A tissue under test **T$_k$** is located between two adjacent measuring points **P$_k$** and **P$_{k+1}$** so as to have a thickness **L.** A number (n-1) of tissues under test **T$_1$** through **T$_{n-1}$** can be sampled from a number n of measuring points **P$_1$** through **P$_n$.**

The thickness variation **Hk(t),** representing the magnitude of shrinkage or dilation of the tissue under test **Tk,** can be calculated as Hk(t)=hk+1(t)-hk(t) based on the magnitudes of displacements **hk(t)** and **hk + 1(t)** of the measuring points **Pk** and **Pk+1.**

The thickness of the tissue **Tk** of the vascular wall **64** changes as the blood pressure changes with the palmus. And such a variation in thickness recurs almost every cardiac cycle. That is why the elastic property is also preferably measured every cardiac cycle (i.e., every time the heart beats). The maximum and minimum values are extracted from the thickness variation **Hk(t)** within one cardiac cycle and the difference between the maximum and minimum values is used as the greatest thickness difference Δ**hk**. Also, the difference between the highest and lowest blood pressures is used as a pulse pressure Δ**p**. If the maximum thickness of the tissue under test is Hm, the strain **Sk** and elastic property χ**k** can be given by:

$$\mathrm{Sk} = \Delta \mathrm{hk}/\mathrm{Hm}$$

$$\chi\mathrm{k} = \Delta \mathrm{p}/\mathrm{Sk} = \Delta \mathrm{p} \cdot \mathrm{Hm}/\Delta \mathrm{hk}$$

The number of the measuring points **Pn** and their interval may be set arbitrarily according to the purpose of the measurements or the property of the vital tissue as the object of measurements. In the example described above, the thickness variation or the elastic property is calculated between two adjacent measuring points. However, the thickness variation or the elastic property may also be calculated between two points that interpose one measuring point between them. In that case, the magnitude of displacement between the two points is preferably the average of the magnitudes of displacements among those two points and their intermediate measuring point.

The thickness variation or the elastic property may be evaluated at one point between two arbitrary points. However, the ultrasonic probe 13 for use in this preferred embodiment has an array of ultrasonic vibrators, and therefore, can evaluate the elastic property at every point within an arbitrary area of the given cross-sectional plane. In this case, the operator can define an arbitrary area by specifying an ROI (= region of interest).

The ROI is shown to allow the operator to define the area in which the elastic property should be measured. And the size and position of the ROI can be freely specified by way of the interface section (not shown) of the ultrasonic diagnostic apparatus 11 while being checked on the display section 21.

The frame calculating section maps the greatest thickness difference, strain or elastic property that has been obtained in this manner from the vital tissue, thereby outputting a spatial distribution of the shape measured values or property measured values as a spatial distribution frame to the display section **21** every cardiac cycle. The spatial distribution frame may be one-dimensional, two-dimensional or even three-dimensional. FIG. **6(a)** schematically illustrates the vascular wall **40** and ROI **41** that are presented on the display section **21**. The image of the vascular wall **40** can be generated by modulating the received signal with a luminance associated with the amplitude or intensity differently from the calculations described above. FIG. **6(b)** shows the elastic property of the vascular wall **40** in the area defined by the ROI **41**. In the area defined by the ROI **41,** frame data items $f(k)_{11}$ through $f(k)_{65}$, which have been mapped to make a matrix of six rows and five columns, are arranged, thereby forming a spatial distribution frame **Fk.** As described above, the frame data items $f(k)_{11}$ through $f(k)_{65}$ represent the shape measured value (e.g., the greatest thickness difference) or the property measured value (e.g., strain or elastic property) of a vital tissue.

The frame calculating section **34** outputs the spatial distribution frame **Fk** to the computed data storage section **20** and gets the frame stored there, or outputs the frame to the display section **21,** which presents the spatial distribution frame **Fk** received. In FIG. **6(b),** the elastic properties are shown in gradations representing their values. Alternatively, the distribution of the elastic properties may be presented as a two-dimensional color image using a number of colors representing the elastic property values in the frame.

Since the greatest thickness difference, strain or elastic property of the vital tissue is calculated every cardiac cycle as described above, the frame data items $f(k)_{11}$ through $f(k)_{65}$ and the spatial distribution frame **Fk** are also updated every cardiac cycle.

Data about the greatest thickness difference, strain and elastic property that have been figured out by the frame calculating section **34** may be stored in, and readily read out from, the computed data storage section **20** as long as the space is left there. For example, if an element such as a ring memory is used as the computed data storage section **20,** the data can always be updated into the newest one and then stored there. Thus, the various sorts of data that has been stored in the computed data storage section **20** can be presented on the display section **21** anytime when necessary.

The difference calculating section **33** calculates differences between the shape measured values or property measured values of two spatial distribution frames that are selected from multiple spatial distribution frames every cardiac cycle. More specifically, the difference calculating section **33** receives the newest spatial distribution frame **Fk** from the frame calculating section **34** or the computed data storage section **20** and calculates a root means square (RMS) of the differences between that newest frame **Fk** and the previous spatial distribution frame **Fk-1** stored in the computed data storage section **20,** thus obtaining a difference **dk**. That is to say, the difference calculating section **33** performs the following calculation:

[Equation 1]

$$dk = \sqrt{\frac{\displaystyle\sum_{i=0,j=0}^{i=m-1,j=n-1}\left(f(k)_{ij} - f(k-1)_{ij}\right)^2}{m \times n}}$$

The difference **dk** thus obtained is output to, and stored in, the computed data storage section **20,** and is also presented on the display section **21**. When one cardiac cycle ends, the frame calculating section **34** calculates the newest spatial distribution frame **Fk+1** and the difference calculating section **33** calculates the difference **dk+1** between the newest frame **Fk+1** and the previous spatial distribution frame **Fk** and outputs the difference to the computed data storage section 20 and also presents the difference **dk+1** on the display section **21**. In this manner, every time a new spatial distribution frame **Fn** is obtained, the difference **dn** is calculated between the new frame **Fn** and the previous spatial distribution frame **Fn-1**. The value of the difference **dn** is updated and presented along with the spatial distribution frame **Fn** on the display section **21** every cardiac cycle.

The difference **dn** is the RMS of the difference between two consecutive frames. The more stabilized the measurements, the smaller the difference **dn.** That is to say, the difference **dn** is an estimate indicating the degree of stability of measurements. That is why the operator of the ultrasonic diagnostic apparatus **11** may check the value of the difference **dn** presented and use it as a reference for stabilizing the measurements while operating the ultrasonic probe **13.**

The difference **dn** does not always have to be presented on the display section **21** as a numerical value. For example, the difference calculating section **33** may generate and present image information representing the magnitude of the difference **dn.** More specifically, if the difference **dn** is relatively big, a moving picture showing a waveform with a large amplitude or a high frequency or a big figure with an arbitrary shape may be generated.

On the other hand, if the difference **dn** is relatively small, a moving picture showing a waveform with a small amplitude or a low frequency or a small figure with an arbitrary shape may be generated. When such a moving picture is presented on the display section **21,** the figure or the waveform presented as the moving picture decreases its size, amplitude or frequency as the difference **dn** decreases. Consequently, the operator can sense the variation in difference **dn** more intuitively than the situation where a numerical value is presented on the display section **21.**

Other than that, the luminance, color tone, length, size, number of pieces, angle or shape of the figure, drawing, or character may be changed with the magnitude of the difference **dn.** Also, not just a moving picture but also a still picture may be generated to represent the difference **dn.**

Optionally, the difference calculating section **33** may generate audio information representing the magnitude of the difference **dn** and output the audio information generated through an audio converter **31** such as a loudspeaker included in the ultrasonic diagnostic apparatus **11** for converting an electrical signal into a sound. For example, if the difference **dn** is relatively big, a sound with a high frequency may be produced. On the other hand, if the difference **dn** is relatively small, a sound with a low frequency may be produced. If such audio information is output through the audio converter **31,** the frequency of the sound output through the audio converter **31** decreases as the difference **dn** narrows. Alternatively, a piezoelectric buzzer may be used as the audio converter **31** such that the period of the buzzing sound to be output discontinuously may be changed with the magnitude of the difference **dn.**

If the variation in the difference **dn** is either presented as image information that can be easily sensed intuitively or output as audio information that is audibly perceivable, the operator can concentrate his or her attention on collecting various other sorts of information, including the position of the probe, the elastic property and other types of measuring information presented on the display section, and the status of the subject, while taking measurements using the ultrasonic diagnostic apparatus **11.**

It should be noted that if the spatial distribution frame presented on the display section **21** includes an area in which there is no need to calculate the shape measured values or property measured values, the difference calculating section **33** may extract only the area in which the shape measured values or property measured values need to be calculated from the spatial distribution frame and calculate the difference. For example, if the ROI **41** includes a vital tissue area other than the vascular wall **40** as shown in FIG. **6(a)** and if the shape measured values or property measured values need to be calculated only in the vascular wall **40,** only the frame data associated with the vascular wall area may be extracted from the spatial distribution frame and the difference may be calculated in the vascular wall tissue. To extract the vascular wall area from the spatial distribution frame, the difference in acoustic impedance may be used, for example. Alternatively, if the ROI **41** includes a vital tissue area other than the vascular wall **40** as shown in FIG. **6(a)** and if the shape measured values or property measured values need to be calculated only in the vascular wall **40,** the ROI **41** may be modified so as to include only the vascular wall **40.**

Also, the difference **dn** does not have to be calculated as the RMS but may also be calculated as the average, the average of the absolute values, the sum, the sum of the absolute values, the variance, the standard deviation or the difference between the maximum and minimum values of the differences. The smaller the difference **dn** obtained by any of these calculations, the less variable the shape measured values or property measured values of the spatial distribution frame and the more stabilized the measurements. Alternatively, a calculating method in which the greater the difference, the less variable the shape measured values or property measured values may also be adopted. For example, the inverse number of the value obtained as a result of any of these calculations may be used as the difference **dn.**

Also, the stability of measurements may be evaluated using a number of the differences **dn.** After the frame calculating section **34** has calculated the newest spatial distribution frame **Fk,** the difference calculating section **33** reads a number (N-1) of consecutive data items of the previous frame **Fk-1** (that was presented one cardiac cycle ago) to another earlier frame **Fk-(N-1)** that was presented (N-1) cardiac cycles ago from the computed data storage section **20.** Next, the difference calculating section **33** calculates the RMS of the frame data differences between each pair of adjacent frames in the N consecutive data items of the newest frame **Fk** to the frame **Fk-(N-1)** and uses them as differences **dk, dk-1, dk-2,** ..., and **dk-(N-2).** Then, the difference calculating section **33** calculates the average of the (N-1) differences and regards the average as the characteristic quantity **Dk** of the differences, which is stored in the computed data storage section **20** and also presented on the display section **21.** The difference calculating section **33** repeatedly performs these operations every cardiac cycle (i.e., every time the newest spatial distribution frame is updated).

As described above, the characteristic quantity **Dk** may be presented on the display section **21** either as a numerical value as it is or as image information such as a moving picture or a still picture of a figure or a drawing representing the magnitude of the characteristic quantity **Dk.** Alternatively, audio information representing the magnitude of the characteristic quantity **Dk** may be generated and output through the audio converter **31.**

The preferable range of the number **N** of differences **dn** that are used to calculate the characteristic quantity **Dk** changes depending on how long the operator and subject can maintain their stabilized posture. For example, when the carotid artery of a human body is measured, N preferably falls within the range of two to six.

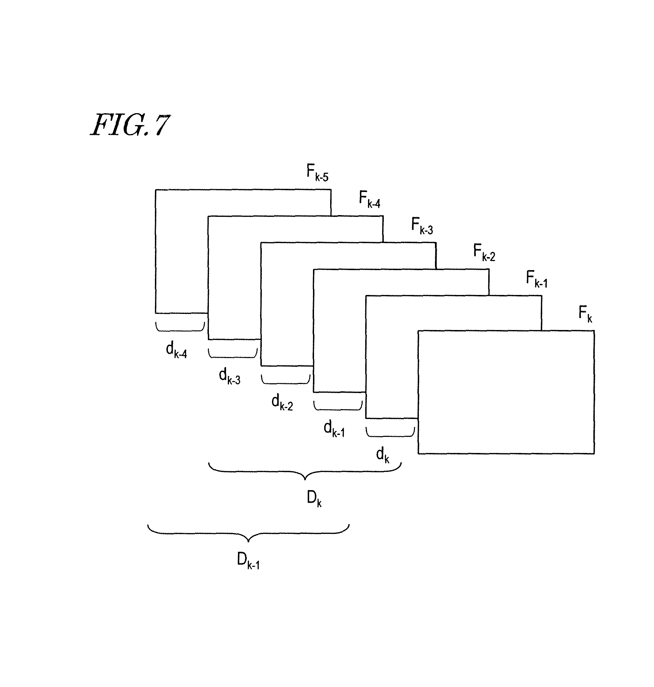

As an example, a situation where N = 5 will be described with reference to FIG. **7.** After the frame calculating section **34** has calculated the newest spatial distribution frame **Fk,** the difference calculating section **33** reads four consecutive data items of the previous frame **Fk-1** (that was presented one cardiac cycle ago) to another earlier frame **Fk-4** that was presented four cardiac cycles ago from the computed data storage section **20.** Next, the difference calculating section **33** calculates the RMS of the frame data differences between each pair of adjacent frames in the five consecutive data items of the newest frame **Fk** to the frame **Fk-4** and uses them as differences **dk, dk-1, dk-2** and **dk-3.** Then, the difference calculating section **33** calculates the average of these four differences and regards the average as the characteristic quantity **Dk** of the differences. As shown in FIG. **7,** the characteristic quantity **Dk-1** when the spatial distribution frame **Fk-1** was obtained one cardiac cycle ago is calculated based on the differences **dk-1, dk-2, dk-3** and **dk-4.** In this manner, every time the newest spatial distribution frame is updated, the characteristic quantity **Dk** is also updated.

The more stabilized the measurements, the smaller the characteristic quantity **Dn** of the differences thus obtained. That is to say, the characteristic quantity **Dn** of the differences also indicates the degree of stability of measurements. That is why the operator of the ultrasonic diagnostic apparatus **11** of the present invention may check the value of the characteristic quantity **Dn** of differences presented and use it as a reference for stabilizing the measurements while operating the ultrasonic probe **13.** In this case, the difference **dn** may also be presented on the display section **21.**

Also, the characteristic quantity **Dn** of differences does not have to be calculated as the average of multiple differences **dn** but may also be calculated as the sum, the variance, the standard deviation, the RMS or the difference between the maximum and minimum values of the differences. As already described for the differences **dn,** the smaller the characteristic quantity **Dn** obtained by any of these calculations, the less variable the measured values. Alternatively, a calculating method in which the greater the difference, the less variable the measured values may also be adopted.

Also, the differences to figure out the characteristic quantity do not have to be calculated between two adjacent or consecutive spatial distribution frames. For example, in calculating the characteristic quantity of differences using five consecutive spatial distribution frames **Fk-4** through **Fk,** the difference **d'k** between Fk and Fk-1, the difference **d'k-1** between Fk and Fk-2, the difference **d'k-2** between Fk and Fk-3, and the difference **d'k-3** between Fk and Fk-4 may be calculated and the average **D'k** of these four differences **d'k** through **d'k-3** may also be calculated.

In the preferred embodiment described above, the two-dimensional distribution of the elastic property of a vascular wall is figured out. Alternatively, the ultrasonic diagnostic apparatus of the present invention is also effectively applicable for use in other circulatory organs such as heart and in liver, mamma and other body tissues.

Also, the preferred embodiment described above is an ultrasonic diagnostic apparatus that figures out the two-dimensional distribution of shape property values or property measured values and presents it as a frame every cardiac cycle. Alternatively, a three-dimensional distribution of shape property values or property measured values may be figured out by using a 3D mechanical probe, for example, and presented as a frame every cardiac cycle.

(EMBODIMENT 2)

Hereinafter, an ultrasonic diagnostic apparatus that presents spatial distribution frames using either the differences **dn** or the characteristic quantity **Dn** of the differences as already described in detail for the first preferred embodiment and a method for controlling such an apparatus will be described as a second preferred embodiment of the present invention. The method of calculating the differences **dn** or the characteristic quantity **Dn** of the differences is just as already described for the first preferred embodiment. Also, although not described again, the ultrasonic diagnostic apparatus of the second preferred embodiment has the same configuration as the counterpart of the first preferred embodiment.

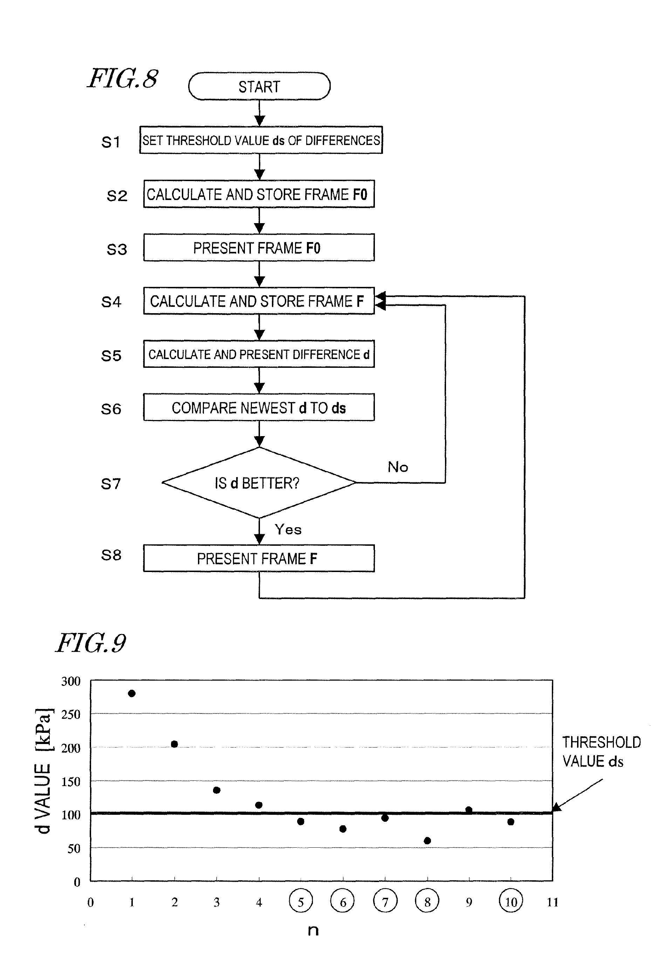

FIG. **8** is a flowchart showing an exemplary procedure of controlling the ultrasonic diagnostic apparatus **11** using the differences **dn.** In FIG. **8,** shown is a method of controlling the presentation of spatial distribution frames based on the result of comparison between the differences **dn** calculated by the difference calculating section **33** and the threshold value **ds** of differences that has been set in advance by the operator of the ultrasonic diagnostic apparatus **11.** The procedure to be described below may be stored as a computer executable program or a piece of firmware on a ROM or any other storage medium provided for the ultrasonic diagnostic apparatus **11.**

First, before starting measurements, the operator sets the threshold value **ds** of differences and enters it into the ultrasonic diagnostic apparatus **11** (in Step **S1).** More specifically, ds is the threshold value of the RMS of differences between two consecutive spatial distribution frames.

Next, the operator handles the ultrasonic diagnostic apparatus **11** and gets the shape measured values or property measured values of a desired area (e.g., a spatial distribution frame **F** that represents the spatial distribution of elastic property) calculated by the frame calculating section **34** and gets the frame **F** stored in the computed data storage section **20** (in Step **S2)** as already described in detail for the first preferred embodiment. The spatial distribution frame **F** calculated in this processing step will be identified herein by F0 because this is the first frame after the measurements have been started. Furthermore, the frame calculating section **34** gets the frame **F0** presented on the display section **21** (in Step S3).

In the next cardiac cycle, the frame calculating section **34** calculates a frame **F1** and gets it stored in the computed data storage section **20** (in Step **S4)**. Also, the frame calculating section **34** calculates a difference **d1** between the frame **F0** stored in the computed data storage section **20** and the frame **F1** just calculated and gets it presented on the display section **21** (in Step **S5).**

The difference calculating section **33** compares the difference **d1** to the threshold value **ds** (in Step **S6),** and determines whether or not the difference **d1** just calculated indicates a higher degree of stability of measurements than the threshold value **ds.** More specifically, the difference calculating section **33** determines whether the RMS is smaller than the threshold value **ds** or not (in Step **S7).** If the difference **d1** is smaller than the threshold value **ds,** the difference calculating section **33** gets the frame **F1** presented on the display section **19** (in Step **S8)** to end the operation in this cardiac cycle. Then, the process goes back to the processing step **S4** to repeat the same processing steps **S4** through **S7** all over again.

It should be noted that in comparing the difference **dn** to the threshold value **ds** in the processing step **S7**, the degree of stability of measurements may be estimated sometimes high and sometimes low depending on how the difference **dn** has been defined. That is to say, according to the definition of the difference **dn,** the degree of stability of measurements may be high when the difference **dn** is greater than the threshold value **ds**.

If the difference **d1** is greater than the threshold value **ds,** the computing section **19** ends the operation for this cardiac cycle, and the process goes back to the processing step **S4** without presenting the newest spatial distribution frame to perform the same processing steps **S4** through **S7** all over again.

If the operator wants to stop or end the measurements, he or she may input a freeze signal to the ultrasonic diagnostic apparatus **11**. The freeze signal may be input at any of the processing steps shown in FIG. **8**. On sensing that the freeze signal has been input, the ultrasonic diagnostic apparatus **11** stops all measurements immediately. On the display section **21,** presented are the last one **F** of the frames showing that the difference d ensure a higher degree of stability than the threshold value **ds** and that difference **d.** To perform such an operation, in Step **S8,** the frame **F** needs to be presented and the frame **F** and the difference **d** at that time need to be stored in the computed data storage section **20.**

FIG. **9** is a graph showing the differences **dn** that were calculated by the ultrasonic diagnostic apparatus of this preferred embodiment every cardiac cycle. The abscissa represents the number of times the spatial distribution frames have been generated since the measurements were started, i.e., the number of cardiac cycles since the beginning of the measurements. The difference **dn** was big for a while after the measurements were started because the position or respiratory state of the operator holding the ultrasonic probe **13** was still not fixed. But the difference **dn** decreased gradually. As indicated by the encircled numbers on the axis of abscissas shown in FIG. **9,** the differences **dn** of the fifth to eighth cardiac cycles and the tenth cardiac cycle are smaller than the threshold value **ds**. The ultrasonic diagnostic apparatus **11** presents the spatial distribution frame on the display section **21** when the difference is smaller than the threshold value **ds**. Specifically, no spatial distribution frames are presented on the display section **21** from the beginning of the measurements through the fourth cardiac cycle. And a spatial distribution frame **F5** is presented for the first time in the fifth cardiac cycle. Thereafter, an updated spatial distribution frame is presented every cardiac cycle through the eighth cardiac cycle. The difference **dn** of the ninth cardiac cycle is greater than the threshold value **ds**. Thus, in the ninth cardiac cycle, the spatial distribution frame is not updated but the previous spatial distribution frame **F8** is presented continuously. After that, the frame is refreshed again into the spatial distribution frame **F10** in the tenth cardiac cycle.

As described above, according to this preferred embodiment, the difference **d** calculated by the difference calculating section **33** is compared to the threshold value **ds** that has been set in advance by the operator and a frame **F** is presented only when the difference **d** is smaller than the threshold value **ds**. Thus, the operator can selectively view only results of measurements that have a certain degree of stability and can make an even more accurate diagnosis.

In the preferred embodiment described above, a control method using the differences **dn** has been described in detail. Alternatively, an ultrasonic diagnostic apparatus that compares the characteristic quantity **Dn** of differences to a preset threshold value **Ds** and controls the presentation of spatial distribution frames based on the result of the comparison can also be realized. In controlling the presentation of spatial distribution frames using the characteristic quantity **Dn** of differences, the processing step S2 of the flowchart shown in FIG. **8** is carried out five times to calculate five differences **d0** through **d4** and then the average of the differences **d0** through **d4** is calculated to obtain the first characteristic quantity **D4** of differences, unlike the control method using the differences **dn.** In that case, in the five cardiac cycles before D4 is obtained, the frame **Fn** may or may not be presented every time the processing step **S3** is performed. To show the operator that the measurements are carried on, the frame **Fn** is preferably presented.

Optionally, the end of the measurements may be controlled by using either the difference dn or the characteristic quantity Dn of differences. For example, a value showing that the results of measurements have sufficient stability may be set as the threshold value $d''s$ or $D''s$ and it is determined whether the difference dn or the characteristic quantity Dn of differences shows a higher degree of stability of measurements than the threshold value $d''s$ or $D''s$ or not. If the answer is YES, the measurements are finished and the last spatial distribution frame is either printed out or stored on a storage medium. This control technique may be combined with the method of controlling the presentation of the spatial distribution frame described above. If the spatial distribution frame is also presented, then the threshold value $d''s$ or $D''s$ for use to control the end of the measurements preferably shows a higher degree of stability of measurements than the threshold value ds or Ds for use to present the spatial distribution frame. Then, the measurements can be finished automatically and a desired spatial distribution frame can be generated when the stability of measurements reaches a sufficiently high level after the measurements were started.

(EMBODIMENT 3)

Hereinafter, an ultrasonic diagnostic apparatus that presents spatial distribution frames using either the differences dn or the characteristic quantity Dn of the differences as already described in detail for the first preferred embodiment and a method for controlling such an apparatus will be described as a third preferred embodiment of the present invention as in the second preferred embodiment. The method of calculating the differences dn or the characteristic quantity Dn of the differences is just as already described for the first preferred embodiment. Also, although not described again, the ultrasonic diagnostic apparatus of the third preferred embodiment has the same configuration as the counterpart of the first preferred embodiment.

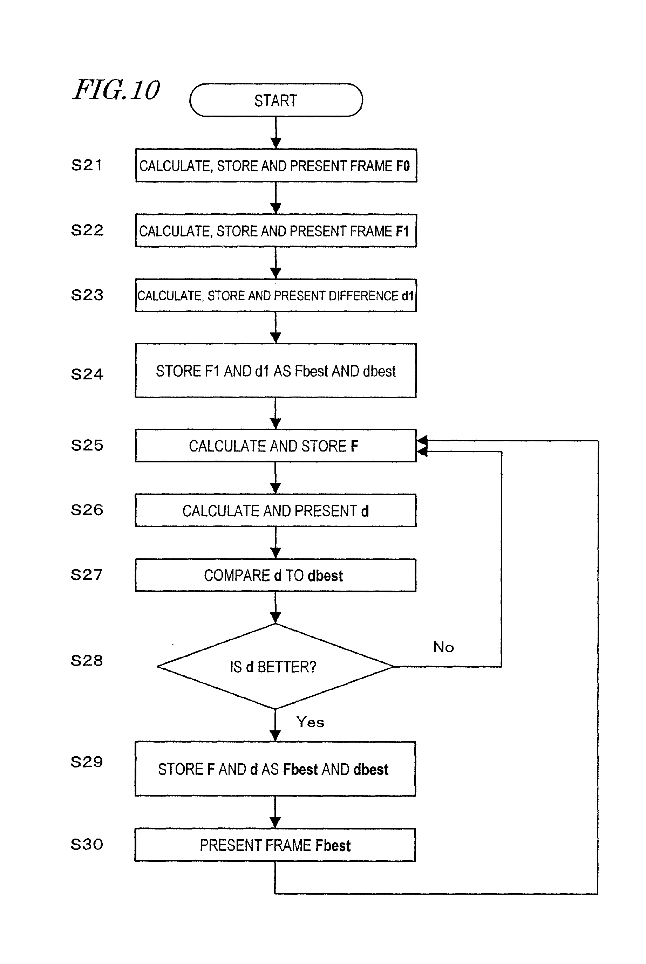

FIG. 10 is a flowchart showing an exemplary procedure of controlling the ultrasonic diagnostic apparatus 11 using the differences dn. In FIG. 10, shown is a method of controlling the presentation of spatial distribution frames based on the result of comparison between the differences dn calculated by the difference calculating section 33 and the threshold value ds of differences that has been set in advance by the operator of the ultrasonic diagnostic apparatus 11.

First, the operator handles the ultrasonic diagnostic apparatus 11 and gets the shape measured values or property measured values of a desired area (e.g., a spatial distribution frame F that represents the spatial distribution of elastic property) calculated by the frame calculating section 34 and gets the frame F stored in the computed data storage section 20 (in Step S21) and presented on the display section 21 as already described in detail for the first preferred embodiment. The spatial distribution frame F calculated in this processing step will be identified herein by F0 because this is the first frame after the measurements have been started.

In the next cardiac cycle, the frame calculating section 34 calculates a spatial distribution frame **F1** and gets it stored in the computed data storage section **20** and presented on the display section **21** (in Step **S22)**. Also, the frame calculating section **34** calculates a difference **d1** between the spatial distribution frame **F0** stored in the computed data storage section 20 and the spatial distribution frame **F1** just calculated and gets it presented on the display section **21** (in Step **S23).**

The difference calculating section 33 stores the frame **F1** and the difference **d1** as the best values $F_{best}$ and $d_{best}$ at that time (in Step **S24).**

In the next cardiac cycle, the frame calculating section **34** calculates a frame **F2** and gets it stored in the computed data storage section **20** (in Step **S25)**. Also, the frame calculating section 34 calculates a difference **d2** between the frame **F1** stored in the computed data storage section 20 and the frame **F2** just calculated and gets it presented on the display section 21 (in Step **S26)**.

The difference calculating section 34 compares the difference **d2** to its best value $d_{best}$ (in Step **S27)**, and determines whether or not the difference **d2** just calculated indicates a higher degree of stability of measurements than the best value $d_{best}$. More specifically, the difference calculating section **33** determines whether the RMS is smaller than $d_{best}$ or not (in Step **S28)**. If the difference **d2** is smaller than $d_{best}$ (i.e., if the difference **d2** guarantees a higher degree of stability of measurements than $d_{best}$), the difference calculating section **33** stores the frame **F2** and the difference **d2** as new best values $F_{best}$ and $d_{best}$ (in Step **S29)** and gets the frame $F_{best}$ presented on the display section **19** (in Step S30) to end the operation for this cardiac cycle. Then, the process goes back to the processing step S25 to repeat the same processing steps S25 through S28 all over again.

If the difference **d2** is greater than $d_{best}$, the difference calculating section **33** ends the operation for this cardiac cycle, and the process goes back to the processing step **S25** without presenting the newest spatial distribution frame to perform the same processing steps **S25** through **S28** all over again.

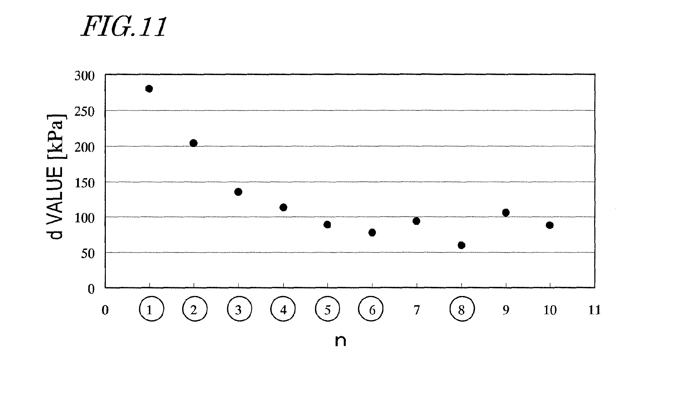

FIG. **11** is a graph showing the differences **dn** that were calculated by the ultrasonic diagnostic apparatus of this preferred embodiment every cardiac cycle. As in FIG. **9,** the abscissa represents the number of times the spatial distribution frames have been generated since the measurements were started, i.e., the number of cardiac cycles since the beginning of the measurements. The difference **dn** was big for a while after the measurements were started because the position or respiratory state of the operator holding the ultrasonic probe **13** was still not fixed. But the difference **dn** decreased gradually. In the cardiac cycles indicated by the encircled numbers on the axis of abscissas shown in FIG. **11,** the best values $F_{best}$ and $d_{best}$ were updated, and therefore, updated spatial distribution frames were presented. That is to say, right after the measurements have been started, the difference **dn** goes on decreasing one cardiac cycle after another and the spatial distribution frame is updated every cardiac cycle. The measurements will get settled soon and the difference **dn** will become substantially constant. Then, the spatial distribution frame is updated only if the difference **dn** shows an even higher degree of stability of measurements.

As described above, according to this preferred embodiment, right after the measurements have been started, the spatial distribution frames are frequently updated and presented. However, as the measurements get settled, the spatial distribution frame that guarantees the highest degree of stability is maintained. Thus, the operator can selectively view only results of measurements that ensure a high degree of stability and can make an even more accurate diagnosis.

In this preferred embodiment, a similar control can also be performed by using the characteristic quantity **Dn** of differences instead of the difference **dn.** In controlling the presentation of spatial distribution frames using the characteristic quantity **Dn** of differences, the processing step **S23** of the flowchart shown in FIG. **10** is carried out five times to calculate five differences **d0** through **d4** and then the average of the differences **d0** through **d4** is calculated to obtain the first characteristic quantity **D4** of differences, unlike the control method using the differences **dn.** In that case, in the five cardiac cycles before D4 is obtained, the frame **Fn** may or may not be presented every time the processing step **S23** is performed. To show the operator that the measurements are carried on, the frame **Fn** is preferably presented.

**INDUSTRIAL APPLICABILITY**

The ultrasonic diagnostic apparatus of the present invention can be used effectively to accurately evaluate the attribute and shape properties of not only a vascular wall but also any other circulatory organ tissue like the heart, the liver, the mamma or any other vital tissue. Also, the ultrasonic diagnostic apparatus is particularly effective in allowing the doctor to make an accurate diagnosis of the shape and property of the given vital tissue.

**Claims**

1. An ultrasonic diagnostic apparatus comprising:

a transmitting section for driving an ultrasonic probe that sends out an ultrasonic transmitted wave toward a tissue of an organism; a receiving section for amplifying an ultrasonic reflected wave to generate a received signal, the ultrasonic reflected wave being produced by getting the ultrasonic transmitted wave reflected by the tissue of the organism and being received by the ultrasonic probe; a frame calculating section for calculating shape measured values of the tissue based on the received signal and figuring out a spatial distribution frame, representing the spatial distribution of the shape measured values and/or property measured values of the organism every cardiac cycle, based on the shape measured values of the tissue; a difference calculating section for calculating a difference between the shape measured values or the property measured values of two frames that have been selected from the spatial distribution frames calculated every cardiac cycle; a storage section for storing at least one of the shape measured values, the property measured values and the difference; and a display section for presenting the spatial distribution frames thereon.

2. The ultrasonic diagnostic apparatus of claim 1, wherein the difference calculating section calculates the difference between the newest and previous spatial distribution frames.

3. The ultrasonic diagnostic apparatus of claim 1, wherein the difference calculating section calculates a number (N-1) of differences between the newest spatial distribution frame and previous (N-1) consecutive spatial distribution frames and further calculates one characteristic quantity, representing the degree of variation among a number N of spatial distribution frames, based on the (N-1) differences.

4. The ultrasonic diagnostic apparatus of claim 3, wherein the difference calculating section calculates the difference between two spatial distribution frames that are continuous with each other on the time axis.

5. The ultrasonic diagnostic apparatus of claim 4, wherein the difference calculating section updates the characteristic quantity every cardiac cycle.

6. The ultrasonic diagnostic apparatus of claim 1, wherein the display section presents the difference.

7. The ultrasonic diagnostic apparatus of claim 3, wherein the display section presents at least one of the difference and the characteristic quantity.

8. The ultrasonic diagnostic apparatus of claim 6, wherein the difference calculating section generates image information based on the difference and the display section presents the image information thereon.

9. The ultrasonic diagnostic apparatus of claim 1, further comprising an acoustic transducer, wherein the difference calculating section generates audio information based on the difference and the acoustic transducer outputs the audio information.

**10.** The ultrasonic diagnostic apparatus of claim 6, wherein the difference calculating section generates the image information based on at least one of the difference and the characteristic quantity and the display section presents the image information thereon.

**11.** The ultrasonic diagnostic apparatus of claim 3, further comprising an acoustic transducer, wherein the difference calculating section generates audio information based on at least one of the difference and the characteristic quantity and the acoustic transducer outputs the audio information.

**12.** The ultrasonic diagnostic apparatus of claim 5, wherein the difference calculating section compares either the difference or the characteristic quantity to a predetermined value, and wherein according to a result of the comparison, the frame calculating section updates the spatial distribution frame to be presented on the display section.

**13.** The ultrasonic diagnostic apparatus of claim 5, wherein the difference calculating section finds a difference or a characteristic quantity, showing the smallest variation between spatial distribution frames, and the display section presents the spatial distribution frame associated with the difference or the characteristic quantity that has been found.

**14.** The ultrasonic diagnostic apparatus of one of claims 1 to 13, wherein the difference is at least one of the average, the average of the absolute values, the sum, the sum of the absolute values, the variance, the standard deviation, the root mean square, and the difference between the maximum and minimum values of either the shape measured values or the property measured values in two frames that have been selected from the multiple spatial distribution frames.

**15.** The ultrasonic diagnostic apparatus of one of claims 3 to 5, wherein the characteristic quantity is at least one of the average, the sum, the variance, the standard deviation, the root mean square, and the difference between the maximum and minimum values of the (N-1) differences.

**16.** The ultrasonic diagnostic apparatus of one of claims 1 to 15, wherein the shape measured value represents a variation in the maximum thickness of the organism's tissue.

**17.** The ultrasonic diagnostic apparatus of one of claims 1 to 15, wherein the property measured value is at least one of the strain and the elastic property of the organism's tissue.

**18.** A method for controlling an ultrasonic diagnostic apparatus using a control section of the apparatus, the method comprising the steps of:

(a) sending out an ultrasonic transmitted wave from an ultrasonic probe and receiving an ultrasonic reflected wave to generate a received signal, the ultrasonic reflected wave being produced by getting the ultrasonic transmitted wave reflected by a tissue of an organism;
(b) calculating shape measured values of the tissue based on the received signal and figuring out a spatial distribution frame, representing the spatial distribution of the shape measured values and/or property measured values of the organism every cardiac cycle, based on the shape measured values of the tissue;
(c) calculating a difference between the shape measured values or the property measured values of two frames that have been selected from the spatial distribution frames calculated every cardiac cycle; and
(d) presenting the spatial distribution frames.

**19.** The method of claim 18, wherein the step (c) includes calculating the difference between the newest and previous spatial distribution frames.

**20.** The method of claim 18, wherein the step (c) includes calculating a number (N-1) of differences between the newest spatial distribution frame and previous (N-1) consecutive spatial distribution frames and further calculating one characteristic quantity, representing the degree of variation among a number N of spatial distribution frames, based on the (N-1) differences.

**21.** The method of claim 20, wherein the step (c) includes calculating the difference between two spatial distribution frames that are continuous with each other on the time axis.

**22.** The method of claim 17, wherein the step (c) includes updating the characteristic quantity every cardiac cycle.

**23.** The method of claim 18, further comprising the step (e1) of presenting the difference.

**24.** The method of claim 20, further comprising the step (e2) of presenting at least one of the difference and the characteristic quantity.

**25.** The method of claim 23, wherein the step (e1) includes generating image information based on the difference and presenting the image information.

**26.** The method of claim 18, further comprising the step (e3) of generating audio information based on the difference and outputting the audio information from an acoustic transducer.

27. The method of claim 24, wherein the step (e2) includes generating the image information based on at least one of the difference and the characteristic quantity and presenting the image information.

28. The method of claim 8, further comprising the step (e4) of generating audio information based on at least one of the difference and the characteristic quantity and outputting the audio information from an acoustic transducer.

29. The method of claim 22, wherein the step (c) includes comparing either the difference or the characteristic quantity to a predetermined value, and wherein the step (d) includes updating the spatial distribution frame to present according to a result of the comparison.

30. The method of claim 22, wherein the step (c) includes finding a difference or a characteristic quantity showing the smallest variation between spatial distribution frames, and
wherein the step (d) includes presenting the spatial distribution frame associated with the difference or the characteristic quantity that has been found.

31. The method of one of claims 18 to 30, wherein the difference is at least one of the average, the average of the absolute values, the sum, the sum of the absolute values, the variance, the standard deviation, the root mean square, and the difference between the maximum and minimum values of either the shape measured values or the property measured values in two frames that have been selected from the multiple spatial distribution frames.

32. The method of one of claims 20 to 26, wherein the characteristic quantity is at least one of the average, the sum, the variance, the standard deviation, the root mean square, and the difference between the maximum and minimum values of the (N-1) differences.

33. The method of one of claims 18 to 32, wherein the shape measured value represents a variation in the maximum thickness of the organism's tissue.

34. The method of one of claims 18 to 32, wherein the property measured value is at least one of the strain and the elastic property of the organism's tissue.

## FIG.1

## FIG.2

*FIG.3*

*FIG.4*

*FIG.5*

P1 ——————— h1(t) ⎫ H1(t) → χ1
     T1
P2 ——————— h2(t) ⎬
     T2 ⎭ H2(t) → χ2
P3 ——————— h3(t)

⋮

Pk-2 ——————— hk-2(t) ⎫
     Tk-2 ⎬ Hk-2(t) → χk-2
Pk-1 ——————— hk-1(t) ⎬
     Tk-1 ⎬ Hk-1(t) → χk-1
Pk ——————— hk(t) ⎬
     Tk ⎬ Hk(t) → χk
Pk+1 ——————— hk+1(t) ⎬
     Tk+1 ⎭ Hk+1(t) → χk+1
Pk+2 ——————— hk+2(t)

⋮

Pn-1 ——————— hn-1(t) ⎫
     Tn-1 ⎬ Hn-1(t) → χn-1
Pn ——————— hn(t)

*FIG.6*   (a)

(b)

*FIG.7*

$F_{k-5}$
$F_{k-4}$
$F_{k-3}$
$F_{k-2}$
$F_{k-1}$
$F_k$

$d_{k-4}$
$d_{k-3}$
$d_{k-2}$
$d_{k-1}$
$d_k$

$D_k$

$D_{k-1}$

*FIG.8*

```
                    ┌─────────────────┐
                    │      START      │
                    └─────────────────┘
                             │
                             ▼
      S1   ┌──────────────────────────────────────────┐
           │ SET THRESHOLD VALUE ds OF DIFFERENCES     │
           └──────────────────────────────────────────┘
                             │
                             ▼
      S2   ┌──────────────────────────────────────────┐
           │ CALCULATE AND STORE FRAME F0             │
           └──────────────────────────────────────────┘
                             │
                             ▼
      S3   ┌──────────────────────────────────────────┐
           │          PRESENT FRAME F0                │
           └──────────────────────────────────────────┘
                             │
                             ▼
      S4   ┌──────────────────────────────────────────┐◄──────┐
           │ CALCULATE AND STORE FRAME F              │       │
           └──────────────────────────────────────────┘       │
                             │                                 │
                             ▼                                 │
      S5   ┌──────────────────────────────────────────┐       │
           │ CALCULATE AND PRESENT DIFFERENCE d        │       │
           └──────────────────────────────────────────┘       │
                             │                                 │
                             ▼                                 │
      S6   ┌──────────────────────────────────────────┐       │
           │        COMPARE NEWEST d TO ds            │       │
           └──────────────────────────────────────────┘       │
                             │                                 │
                             ▼                          No     │
      S7          ╱─────────────────────╲  ────────────────────┤
                  ╲     IS d BETTER?     ╱                      │
                   ╲───────────────────╱                       │
                             │ Yes                             │
                             ▼                                 │
      S8   ┌──────────────────────────────────────────┐       │
           │          PRESENT FRAME F                 │       │
           └──────────────────────────────────────────┘       │
                             │                                 │
                             └─────────────────────────────────┘
```

*FIG.9*

THRESHOLD
VALUE ds

*FIG.10*

```
              ( START )
                  │
                  ▼
S21   ┌─────────────────────────────────────┐
      │ CALCULATE, STORE AND PRESENT FRAME F0 │
      └─────────────────────────────────────┘
                  │
                  ▼
S22   ┌─────────────────────────────────────┐
      │ CALCULATE, STORE AND PRESENT FRAME F1 │
      └─────────────────────────────────────┘
                  │
                  ▼
S23   ┌───────────────────────────────────────────┐
      │ CALCULATE, STORE AND PRESENT DIFFERENCE d1 │
      └───────────────────────────────────────────┘
                  │
                  ▼
S24   ┌─────────────────────────────────────┐
      │   STORE F1 AND d1 AS Fbest AND dbest  │
      └─────────────────────────────────────┘
                  │
                  ▼
S25   ┌─────────────────────────────────────┐◄──────┐
      │        CALCULATE AND STORE F          │       │
      └─────────────────────────────────────┘       │
                  │                                  │
                  ▼                                  │
S26   ┌─────────────────────────────────────┐       │
      │        CALCULATE AND PRESENT d         │       │
      └─────────────────────────────────────┘       │
                  │                                  │
                  ▼                                  │
S27   ┌─────────────────────────────────────┐       │
      │          COMPARE d TO dbest           │       │
      └─────────────────────────────────────┘       │
                  │                                  │
                  ▼                          No      │
S28          ◇ IS d BETTER? ◇ ─────────────────────┘
                  │ Yes
                  ▼
S29   ┌─────────────────────────────────────┐
      │   STORE F AND d AS Fbest AND dbest    │
      └─────────────────────────────────────┘
                  │
                  ▼
S30   ┌─────────────────────────────────────┐
      │         PRESENT FRAME Fbest           │
      └─────────────────────────────────────┘
                  │
                  └──────────────────────────────────┘
```

*FIG.11*

**INTERNATIONAL SEARCH REPORT**

| International application No. |
| --- |
| PCT/JP2005/019088 |

A. CLASSIFICATION OF SUBJECT MATTER
*A61B8/08*(2006.01)

According to International Patent Classification (IPC) or to both national classification and IPC

B. FIELDS SEARCHED

Minimum documentation searched (classification system followed by classification symbols)
*A61B8/08*(2006.01)

Documentation searched other than minimum documentation to the extent that such documents are included in the fields searched

| | | | |
| --- | --- | --- | --- |
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
| --- | --- | --- |
| A | JP 5-168626 A (Aloka Co., Ltd.), 02 July, 1993 (02.07.93), Column 8, lines 14 to 18 (Family: none) | 1-34 |
| A | JP 2000-229078 A (Japan Science and Technology Corp.), 22 August, 2000 (22.08.00), Column 14, lines 13 to 20; column 21, line 15 to column 22, line 11; column 25, lines 34 to 37 (Family: none) | 1-34 |
| A | Hideyuki HASEGAWA et al., Measurement of Local Elasticity of Human Carotid Arterial Walls and Its Relationship with Risk Index of Atherosclerosis, Proc 1998 IEEE Ultrason Symp, 1998.10, pages 1451 to 1454 | 1-34 |

☐ Further documents are listed in the continuation of Box C.     ☐ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 12 January, 2006 (12.01.06) | 24 January, 2006 (24.01.06) |

| Name and mailing address of the ISA/ | Authorized officer |
| --- | --- |
| Japanese Patent Office | |
| Facsimile No. | Telephone No. |

Form PCT/ISA/210 (second sheet) (April 2005)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 10005226 A

**Non-patent literature cited in the description**

- **HIROSHI KANAI et al.** Elasticity Imaging of Atheroma with Transcutaneous Ultrasound Preliminary Study. *Circulation,* 2003, vol. 107, 3018-3021